(19) Europäisches Patentamt / European Patent Office / Office européen des brevets

(11) **EP 2 293 061 A1**

(12) **DEMANDE DE BREVET EUROPEEN**

(43) Date de publication:
**09.03.2011 Bulletin 2011/10**

(51) Int Cl.:
*G01N 33/28* (2006.01)      *G01N 31/12* (2006.01)

(21) Numéro de dépôt: **10290439.8**

(22) Date de dépôt: **04.08.2010**

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**
Etats d'extension désignés:
**BA ME RS**

(30) Priorité: **08.09.2009  FR 0904258**

(71) Demandeur: **IFP Energies Nouvelles**
**92852 Rueil-Malmaison Cedex (FR)**

(72) Inventeurs:
• **Behar, Françoise**
  **75007 Paris (FR)**
• **Haeseler, Frank**
  **92250 La Garenne Colombes (FR)**

(54) **Méthode pour analyser une huile biodégradée par quantification d'hydrocarbures saturés liés**

(57)  - Méthode d'analyse d'un échantillon pétrolier biodégradé par détermination de la quantité d'hydrocarbures saturés liés à la famille d'hydrocarbures aromatiques de plus de quatorze atomes de carbone, à la famille de résines, et à la famille d'asphaltènes.
- On extrait d'un gisement un échantillon pétrolier, et l'on sépare au moins la famille d'hydrocarbures aromatiques de plus de quatorze atomes de carbone, la famille de résines, et la famille d'asphaltènes. On détermine la quantité d'hydrocarbures saturés liés à chacune de ces familles, en générant les hydrocarbures saturés liés à chacune de ces familles au moyen de pyrolyses en milieu fermé. Chacune des trois familles est pyrolysées de façon indépendante, avec des gammes de températures de pyrolyse et des gammes de temps de pyrolyse choisies pour permettre un craquage des famille générant les hydrocarbures saturés initialement liés aux hydrocarbures de chacune des trois familles. Un dosage des hydrocarbures saturés générés par les pyrolyses permet de déterminer la quantité d'hydrocarbures saturés liés à chacune des familles.
- Application à l'exploration et l'exploitation de gisement pétrolier.

Fig. 1

**Description**

La présente invention concerne le domaine de l'industrie pétrolière. Elle concerne une méthode pour évaluer le taux de biodégradation de fluides pétroliers.

La méthode selon l'invention fournit un outil d'évaluation utile notamment aux géologues soucieux d'orienter les investigations hors de zones à risque. En particulier, on peut l'utiliser dans le domaine technique de la modélisation de bassin pour prédire le risque de biodégradation à l'échelle du bassin, et en déduire les propriétés de ces fluides, en particulier son degré API.

En effet, il est couramment reconnu que la biodégradation, définie comme la destruction sélective d'une partie des molécules composant un brut pétrolier par des bactéries, peut se développer jusqu'à des températures pouvant atteindre 70 à 80°C. De telles températures sont courantes en domaine marin profond qui est une des zones où la recherche pétrolière est la plus active actuellement.

Cette biodégradation a pour effet de modifier la qualité de l'huile. Cette qualité est mesurée par la viscosité et le degré API qui est l'inverse de la densité. Elle est fonction de la composition de l'huile (l'échelle de degrés API est entre 0 et 60). Une forte teneur en hydrocarbures légers, augmente le degré API, alors qu'une forte proportion de résines et d'asphaltènes (NSO) fait décroître le degré API. Au cours de l'histoire géologique d'un fluide, la maturation thermique tend à alléger les huiles de réservoir, donc tend à augmenter le degré API, tandis que l'altération microbienne tend à alourdir l'huile, et donc à décroître son degré API.

La biodégradation est un phénomène biologique qui peut affecter la quantité et la composition chimique des huiles de réservoir. Les bactéries, responsables de ces réactions, sont présentes dans le milieu poreux et vivent dans l'eau qui circule dans le milieu poreux. Ces bactéries utilisent les hydrocarbures comme source de carbone et d'énergie pour assurer leur développement et leur maintien.

Dans l'industrie pétrolière, il est très important de connaître le rôle de la biodégradation dans les processus d'altération de l'huile pour connaître la qualité et la quantité d'huile attendue dans un bassin sédimentaire. Pour y parvenir, il faut disposer d'une méthode permettant d'estimer les pertes d'hydrocarbures dans les différentes classes chimiques des huiles susceptibles d'être altérées par biodégradation.

Cette biodégradation, dont l'effet macroscopique est généralement d'alourdir l'huile, d'augmenter sa viscosité et de diminuer son degré API, représente un risque majeur pour les compagnies pétrolières dont les forages en mer profonde représentent un investissement financier important. Toute méthode permettant de réduire ce risque est donc d'un intérêt majeur pour ces compagnies.

On connaît de nombreux travaux permettant de diagnostiquer si un fluide pétrolier a été altéré par biodégradation. On connaît également des échelles de biodégradation qui permettent de classer les fluides pétroliers par ordre de biodégradation croissante ou décroissante. Ces travaux ont été résumés par les trois documents suivants :

- Connan et al., 1979,« Advances in Organic Geochemistry », Pegamon Press Oxford 1-17;

- Peters et Moldowan, 1993, « The biomarker guide », Prentice Hall,

- Peters et al., 2005, « the biomarker guide : biomarkers and isotopes in petroleum exploration and earth history », Cambridge University Press

Ces travaux portent essentiellement sur le suivi de certaines molécules présentes dans les huiles : ces molécules peuvent être des *n*-alcanes linéaires ou ramifiées, des *cyclo* - alcanes de 1 à plusieurs cycles et des molécules aromatiques.

Par ailleurs, lorsque les hydrocarbures saturés qui sont préférentiellement dégradés par biodégradation ne sont plus présents dans les huiles, il est difficile de déterminer l'origine des huiles, et donc de les replacer dans l'histoire géologique du bassin. Pour déterminer l'origine de ces huiles, il a été montré que des aromatiques, résines ou asphaltenes pyrolysés dans certaines conditions pouvaient générer des hydrocarbures dont la distribution générale était proche de celle des hydrocarbures saturés avant biodégradation. Cette distribution peut donc être utilisée pour déterminer l'origine de ces huiles biodégradées.

De façon générale, les méthodes connues visent soit à décrire soit à diagnostiquer un état de biodégradation des huiles et à quantifier très partiellement des pertes pour certaines familles chimiques. Ces familles chimiques sont généralement les hydrocarbures saturés et aromatiques compris dans la gamme de carbone de 5 à 14 atomes, les *n*-alcanes de plus de 14 atomes de carbone, certains *iso*-alcanes comme le pristane et le phytane.

L'objet de l'invention concerne une méthode expérimentale permettant de quantifier les pertes en hydrocarbures, notamment pour l'ensemble des hydrocarbures aromatiques de plus de 14 atomes de carbones, pour l'ensemble de la famille des résines et pour l'ensemble de la famille des asphaltènes.

**La méthode selon l'invention**

L'invention concerne une méthode d'analyse d'un échantillon pétrolier contenant au moins une famille d'hydrocarbures aromatiques de plus de quatorze atomes de carbone, une famille de résines, et une famille d'asphaltènes, dans laquelle on détermine une quantité d'hydrocarbures saturés liés à chacune desdites familles, en réalisant les étapes suivantes :

- on réalise une séparation desdites familles au sein dudit échantillon pétrolier ;

- on génère les hydrocarbures saturés liés à chacune desdites familles au moyen de pyrolyses en milieu fermé, chacune des trois familles étant pyrolysées de façon indépendante, avec des gammes de températures de pyrolyse et des gammes de temps de pyrolyse déterminées pour effectuer un craquage desdites familles générant les hydrocarbures saturés initialement liés aux hydrocarbures de chacune des trois familles ;

- on réalise une séparation des hydrocarbures saturés à la fin de chaque pyrolyse ;

- on détermine la quantité d'hydrocarbures saturés liés à chacune desdites familles en dosant les hydrocarbures saturés ainsi séparés.

Selon l'invention, les gammes de températures de pyrolyse peuvent être comprises entre 300 et 350°C, et les gammes de temps de pyrolyse sont supérieures à une heure. Ces gammes peuvent également être déterminées à partir de paramètres cinétiques (E, A) de réactions chimiques affectant les familles au cours d'un craquage d'une huile, au moyen des étapes suivantes pour chaque famille :

- on détermine une vitesse de dégradation de ladite famille pour une température donnée en utilisant la loi d'Arrhenius, et on en déduit un taux de conversion pour différents temps de pyrolyse ;

- on sélectionne les gammes de températures de pyrolyse et de temps de pyrolyse qui conduisent à un taux de conversion de 100%.

Selon un mode de réalisation, on ne génère qu'une partie des hydrocarbures saturés initialement liés aux hydrocarbures de chacune des trois familles, les gammes de températures de pyrolyse et de temps de pyrolyse sont déterminées à partir de paramètres cinétiques (E, A) de réactions chimiques affectant les familles au cours d'un craquage d'une huile, au moyen des étapes suivantes pour chaque famille :

- on détermine une vitesse de dégradation de ladite famille pour une température donnée en utilisant la loi d'Arrhenius, et on en déduit un taux de conversion pour différents temps de pyrolyse ;

- on sélectionne les gammes de températures de pyrolyse et de temps de pyrolyse qui conduisent à un taux de conversion suffisant pour récupérer des quantités d'hydrocarbures saturés mesurables,

- on calcul, grâce au taux de conversion connus pour les différentes conditions de pyrolyse, la quantité maximale d'hydrocarbures saturés pour 100% de conversion.

De façon préférentielle, le dosage est réalisé en utilisant les classes chimiques suivantes :

| Classe 1 | $CO_2$ |
|----------|--------|
| Classe 2 | $H_2S$ |
| Classe 3 | $C_1$ |
| Classe 4 | $C_2$-$C_4$ |
| Classe 5 | $C_6$-$C_{14}$ *n*-alcanes |
| Classe 6 | $C_6$-$C_{14}$ *iso*-alcanes |
| Classe 6 | $C_6$-$C_{14}$ *cyclo*-alcanes |
| Classe 7 | $C_6$-$C_{14}$ aromatiques |

(suite)

| Classe 8 | $C_{14+}$ *n*-alcanes |
|---|---|
| Classe 9 | $C_{14+}$ *iso*-alcanes |
| Classe 10 | $C_{14+}$ *cyclo*-alcanes |
| Classe 11 | $C_{14+}$ aromatiques |
| Classe 12 | Résines |
| Classe 13 | Asphaltènes |
| Classe 14 | Résidu insoluble |

Selon l'invention, on peut déterminer une fraction biodégradable d'une huile biodégradée extraite d'un gisement pétrolier, en réalisant les étapes suivantes :

- on prélève un échantillon d'huile non biodégradée dans ledit gisement pétrolier ;

- on détermine, pour lesdits échantillon d'huile non biodégradée et biodégradée, la quantité d'hydrocarbures saturés liés à chacune desdites familles, hydrocarbures aromatiques de plus de quatorze atomes de carbone, résines et asphaltènes ;

- on détermine la fraction biodégradable de l'huile biodégradée en calculant le rapport entre la quantité d'hydrocarbures saturés de l'échantillon biodégradé et la quantité d'hydrocarbures saturés de l'échantillon non biodégradé.

Selon l'invention, on peut déterminer un degré API d'une huile biodégradée extraite d'un gisement pétrolier, en réalisant les étapes suivantes :

- on réalise une phase de calibration, au cours de laquelle :

- on prélève au moins deux échantillons d'huile dans ledit gisement pétrolier ;

- on détermine pour chacun desdits échantillons d'huile une quantité d'hydrocarbures saturés liés à chacune desdites familles, hydrocarbures aromatiques de plus de quatorze atomes de carbone, résines et asphaltènes ;

- on détermine pour chacun desdits échantillons d'huile un degré API ;

- on détermine une relation entre lesdites quantité d'hydrocarbures saturés liés et les degrés API ;

- on détermine le degré API de tout échantillon d'huile extrait du gisement pétrolier, en déterminant des quantités d'hydrocarbures saturés liés à chacune desdites familles, hydrocarbures aromatiques de plus de quatorze atomes de carbone, résines et asphaltènes, et en appliquant ladite relation.

Selon l'invention, on peut déterminer une fraction biodégradable d'une huile biodégradée extraite d'un gisement pétrolier, en réalisant les étapes suivantes :

- on réalise une phase de calibration, au cours de laquelle :

- on prélève au moins deux échantillons d'huile dans ledit gisement pétrolier ;

- on détermine pour chacun desdits échantillons d'huile une quantité d'hydrocarbures saturés liés à chacune desdites familles, hydrocarbures aromatiques de plus de quatorze atomes de carbone, résines et asphaltènes ;

- on détermine pour chacun desdits échantillons d'huile un degré API ;

- on détermine une relation entre lesdites quantité d'hydrocarbures saturés liés et les degrés API ;

- on détermine la fraction biodégradable de tout échantillon d'huile extrait du gisement pétrolier, en déterminant le degré API, et en appliquant ladite relation.

D'autres caractéristiques et avantages de la méthode selon l'invention, apparaîtront à la lecture de la description ci-après d'exemples non limitatifs de réalisations, en se référant aux figures annexées et décrites ci-après.

**Présentation succincte des figures**

- La figure 1 illustre les étapes de la méthode expérimentale selon l'invention.

- La figure 2 illustre l'application de la méthode selon l'invention pour déterminer le degré API ou la fraction biodégradable d'une huile.

**Description détaillée de la méthode**

L'objet de l'invention est une méthode expérimentale d'analyse d'un brut pétrolier. Les fluides pétroliers extraits du sous-sol sont des mélanges très complexes de composés organiques. Les principales familles de composés sont les hydrocarbures saturés, les aromatiques, les résines et les asphaltènes. Les hydrocarbures saturés forment le groupe majoritaire sauf dans les pétroles lourds dégradés. Cette famille comporte des *n*-alcanes, mais aussi des *iso*-alcanes et des *cyclo*-alcanes. La famille des hydrocarbures aromatiques contient des composés de 1 à 5 noyaux aromatiques, substitués par des groupements méthyles ou alkyles. Les composés soufrés des pétroles comportent des thiols, des sulfures, des thiophènes, des benzothiophènes, des dibenzothiophènes, des naphtothiophènes, et des bithiophènes. Les résines et les asphaltènes sont des molécules de haute masse moléculaire contenant des hétéroatomes : N, S et O, d'où l'appellation NSO. Le pétrole brut peut contenir des traces de métaux. Le Vanadium peut représenter jusqu'à 75% de la teneur en métaux suivi du Nickel.

Le niveau de biodégradation d'une huile est directement lié à la quantité d'hydrocarbures saturés présents dans l'huile. Les hydrocarbures saturés se présentent sous deux formes : libres, c'est-à-dire ce que l'on appelle la fraction des hydrocarbures saturés dans l'huile, et les hydrocarbures saturés liés aux autres fractions (aromatiques, résines et asphaltènes). On connaît des méthodes pour déterminer la quantité d'hydrocarbures présents dans la fraction des saturés (hydrocarbures saturés libres). La méthode selon l'invention permet de déterminer la quantité d'hydrocarbures saturés liés aux familles d'hydrocarbures aromatiques de plus de 14 atomes de carbone ($C_{14+}$ aromatiques), de résines et d'asphaltènes.

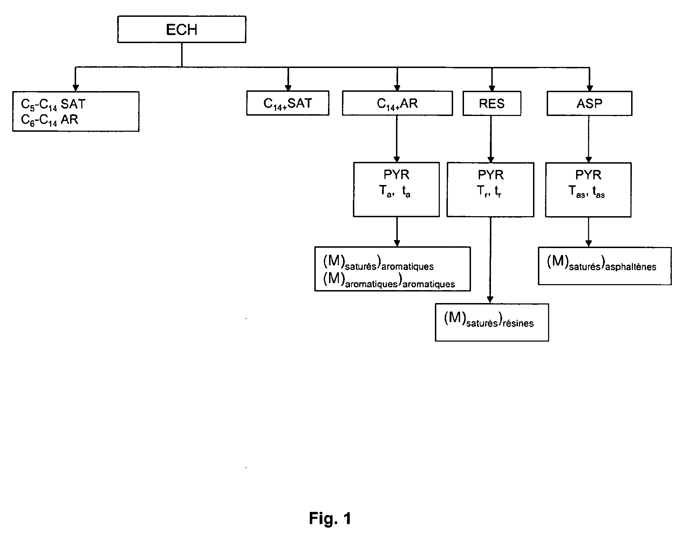

La figure 1 illustre les étapes de la méthode selon l'invention. Après avoir extrait un échantillon (*ECH*) d'huile du sous-sol, on détermine les quantités d'hydrocarbures saturés liés en appliquant les étapes suivantes :

1- On sépare les familles $C_{14+}$ aromatiques, résines et asphaltènes (*SEP1*)

2- On pyrolyse en milieu fermé chacune des trois familles indépendamment (PYR)

3- On sépare et dose, dans les produits de pyrolyse, les hydrocarbures saturés (PES).

1- Séparation des familles $C_{14+}$ aromatiques, résines et asphaltènes (*SEP*)

Les familles $C_{14+}$ aromatiques ($C_{14}$+AR), résines (RES) et asphaltènes (ASP) sont séparées par des techniques classiques et bien connues des spécialistes. Généralement, les asphaltènes sont séparés par précipitation dans l'heptane. La fraction soluble dans l'heptane est séparée en $C_{14+}$ saturés, $C_{14+}$ aromatiques et résines par chromatographie liquide préparative. Après évaporation des solvants utilisés, les quatre familles sont pesées.

Généralement, la séparation conduit également à la séparation des familles $C_{5-14}$ saturés ($C_{5-14}$SAT), $C_{6-14}$ aromatiques ($C_{6-14}$AR), et $C_{14+}$ saturés ($C_{14+}$SAT), comme l'illustre la figure 1.

2- Pyrolyse en milieu fermé des familles $C_{14+}$ aromatiques, résines et asphaltènes *(PYR)*

Chacune des trois familles est ensuite chauffée dans une chambre de pyrolyse fermée en atmosphère de gaz inerte, généralement des tubes en or soudés aux extrémités. Les conditions de pyrolyse sont ajustées de telle sorte à permettre le craquage des familles $C_{14+}$ aromatiques, des résines et des asphaltènes, et à générer les structures saturées qui étaient initialement des fonctions substituées sur les structures aromatiques, résines ou asphaltènes.

Pour ce faire, on détermine deux paramètres : la gamme de température de pyrolyse $T_i$, et la gamme des temps de pyrolyse $t_i$. Pour chacune des trois familles, on effectue plusieurs points de pyrolyse afin de déterminer la conversion desdites familles ainsi que celle des hydrocarbures saturés générés au cours de ces réactions de craquage. Ces conditions de pyrolyse en milieu fermé, réalisée à au moins une températures de $T_a$ °C, pendant au moins un temps $t_a$, permet ainsi le craquage des hydrocarbures saturés initialement liés aux hydrocarbures de la famille $C_{14+}$ aromatiques.

Ces conditions de pyrolyse en milieu fermé, réalisée à au moins une température de $T_r$ °C, pendant au moins un temps $t_r$, permet ainsi le craquage des hydrocarbures saturés initialement liés aux hydrocarbures de la famille résines.

Ces conditions de pyrolyse en milieu fermé, réalisée à au moins une température de $T_{as}$ °C, pendant au moins un temps $t_{as}$, permet ainsi le craquage des hydrocarbures saturés initialement liés aux hydrocarbures de la famille asphaltènes.

Pour définir les conditions de pyrolyse les plus appropriées, on peut utiliser les paramètres cinétiques de craquage proposés dans le document suivant :

- Behar F., et al., 2008, « Elaboration of a new compositional kinetic schema for oil cracking », Organic Geochemistry 39 (2008) 764-782

Ce dernier document présente le tableau suivant, qui donne, entre autre, les paramètres cinétiques (E, A) des réactions chimiques liées au craquage de l'huile, et affectant les classes chimiques présentes dans cette huile :

EP 2 293 061 A1

| | H$_2$S | C$_1$ | C$_2$ | C$_3$-C$_4$ | C$_6$-C$_14$ sat | C$_6$-C$_14$ aro | C$_{14+}$ sat | C$_{14+}$ aro-1 | C$_{14+}$ aro-2 | C$_{14+}$ aro-3 | NSOs | solid residue | Total | H content Meas. | H content Comp. | E kcal/mol | P % | A s$^{-1}$ |
|---|---|---|---|---|---|---|---|---|---|---|---|---|---|---|---|---|---|---|
| NSOs | 0.9 | 1.3 | 1.3 | 1.8 | 7.2 | 4.7 | 6.7 | 49.1 | | | | 27.1 | 100.0 | 9.52 | 9.72 | 50 | 37.4 | 3.16E+12 |
| | | | | | | | | | | | | | | | | 52 | 4.1 | |
| | | | | | | | | | | | | | | | | 54 | 52.8 | |
| | | | | | | | | | | | | | | | | 58 | 5.7 | |
| | | | | | | | | | | | | | | | | Total | 100.0 | |
| C$_{14+}$ aro-1 | 1.3 | 2.3 | 1.9 | 4.7 | 6.4 | 3.2 | 17.8 | | 47.5 | | | 15.0 | 100.0 | 9.94 | 9.83 | 52 | 20.8 | 3.05E+13 |
| | | | | | | | | | | | | | | | | 54 | 79.2 | |
| | | | | | | | | | | | | | | | | Total | 100.0 | |
| C$_{14+}$ aro-2 | | 4.9 | | | | | | | | 63.2 | | 31.9 | 100.0 | | | 47 | | 4.60E+10 |
| C$_{14+}$ sat | | 3.8 | 4.9 | 17.2 | 53.9 | 5.8 | | | 14.4 | | | | 100.0 | 14.93 | 14.96 | 64 | 55.4 | 3.85E+16 |
| | | | | | | | | | | | | | | | | 66 | 25.2 | |
| | | | | | | | | | | | | | | | | 68 | 17.2 | |
| | | | | | | | | | | | | | | | | 70 | 1.8 | |
| | | | | | | | | | | | | | | | | 72 | 0.4 | |
| | | | | | | | | | | | | | | | | Total | 100.0 | |
| C$_5$-C$_{14}$ sat | | 1.7 | 27.8 | 38.3 | | 32.2 | | | | | | | 100.0 | 15.60 | 15.99 | 67.2 | 100.0 | 3.85E+16 |

A partir de ce tableau, on détermine les temps $T_a$ et le temps $t_a$ en appliquant les étapes suivantes :

- connaissant les paramètres cinétiques E et A pour les différentes classes, il est possible de calculer en utilisant la loi d'Arrhenius (k = A e$^{-E/RT}$) la vitesse k de dégradation de ladite classe pour une température donnée, et d'en déduire la conversion pour différents temps de chauffe en utilisant la formule Log (1-conversion)=kt. Ainsi, par exemple, il est possible de déterminer les conditions de pyrolyse pour lesquelles la famille C$_{14+}$ aromatique est susceptible de générer les hydrocarbures saturés et de déterminer la conversion des hydrocarbures saturés dans ces mêmes conditions. Dans ce tableau sont également indiqués les paramètres cinétiques pour l'ensemble de fraction qui contient les résines et les asphaltènes et qui est appelée NSOs. Dans les conditions de pyrolyse sélectionnées, seuls les hydrocarbures saturés contenant plus de 14 atomes de carbone peuvent avoir subi un début de craquage thermique. Aussi, les quantités observées sont corrigées en utilisant les paramètres cinétiques du Tableau.

- ainsi les pyrolyses sont réalisées dans les conditions préalablement sélectionnées sur la famille C$_{14+}$ aromatique, les résines, les asphalténes ou des NSOs (résines+asphaltènes). Un bilan de masse complet est réalisé pour chaque point de pyrolyse.

- puis la quantité observée des hydrocarbures C$_{14+}$ saturés est corrigée de la conversion de ces derniers si elle est observée.

- la quantité totale incluant les hydrocarbures saturés de plus et moins 14 atomes de carbone est déterminée pour plusieurs conversions des C$_{14+}$ aromatiques, ou des résines, ou des asphaltènes, ou des NSOs.

- connaissant les conversions desdites familles, il est possible de calculer la quantité totale d'hydrocarbures saturés générés pour 100% de conversion.

On procède de même pour $T_r$, $t_r$, $T_{as}$ et $t_{as}$.
Les températures sont typiquement comprises entre 300 et 350°C pour des durées de quelques heures à quelques jours. Ces conditions permettent de générer des quantités suffisantes d'hydrocarbures saturés, sans que ces derniers subissent un craquage thermique, ce qui pourrait altérer significativement leur empreinte initiale.
On dispose ainsi de deux modes de réalisations :

Selon un premier mode, les gammes de températures de pyrolyse et de temps de pyrolyse sont déterminées à partir des paramètres cinétiques (E, A) de réactions chimiques affectant les familles au cours d'un craquage d'une huile, au moyen des étapes suivantes pour chaque famille :

- on détermine la vitesse de dégradation de la famille pour une température donnée en utilisant la loi d'Arrhenius, et on en déduit un taux de conversion pour différents temps de pyrolyse ;

- on sélectionne les gammes de températures de pyrolyse et de temps de pyrolyse qui conduisent à un taux de conversion de 100%.

Selon un second mode, on ne génère qu'une partie des hydrocarbures saturés initialement liés aux hydrocarbures de chacune des trois familles, et les gammes de températures de pyrolyse et de temps de pyrolyse sont déterminées à partir de paramètres cinétiques (E, A) de réactions chimiques affectant les familles au cours d'un craquage d'une huile, au moyen des étapes suivantes pour chaque famille :

- on détermine la vitesse de dégradation de la famille pour une température donnée en utilisant la loi d'Arrhenius, et on en déduit un taux de conversion pour différents temps de pyrolyse ;

- on sélectionne les gammes de températures de pyrolyse et de temps de pyrolyse qui conduisent à un taux de conversion suffisant pour récupérer des quantités d'hydrocarbures saturés mesurables,

- on calcul, grâce au taux de conversion connus pour les différentes conditions de pyrolyse, la quantité maximale d'hydrocarbures saturés pour 100% de conversion.

3- Séparation et dosage des hydrocarbures saturés issus de la pyrolyse (*PES*)

Après chaque pyrolyse, le tube en or est ouvert et la quantité d'hydrocarbures saturés générée par pyrolyse est dosée au moyen de méthodes quantitatives appropriées (pesée (bilan massique), chromatographie en phase gazeuse, résonance magnétique nucléaire, spectrométrie de masse ....). De préférence, on utilise les quatorze classes chimiques suivantes :

| Classe 1 | $CO_2$ |
|---|---|
| Classe 2 | $H_2S$ |
| Classe 3 | $C_1$ |
| Classe 4 | $C_2$-$C_4$ |
| Classe 5 | $C_6$-$C_{14}$ *n*-alcanes |
| Classe 6 | $C_6$-$C_{14}$ *iso*-alcanes |
| Classe 6 | $C_6$-$C_{14}$ *cyclo*-alcanes |
| Classe 7 | $C_6$-$C_{14}$ aromatiques |
| Classe 8 | $C_{14+}$ *n*-alcanes |
| Classe 9 | $C_{14+}$ *iso*-alcanes |
| Classe 10 | $C_{14+}$ *cyclo*-alcanes |
| Classe 11 | $C_{14+}$ aromatiques |
| Classe 12 | Résines |
| Classe 13 | Asphaltènes |
| Classe 14 | Résidu insoluble |

La masse des éléments des classes 3 à 10, issue de la pyrolyse des hydrocarbures de la famille $C_{14+}$ aromatiques correspond à la quantité d'hydrocarbures saturés initialement liés aux hydrocarbures de cette famille aromatique. Cette masse est notée, $(M)_{saturés})_{aromatiques}$, elle correspond à la fraction biodégradable issue des $C_{14+}$ aromatiques :

$$(M)_{saturés})_{aromatiques} = m(C_1\text{-}C_4) + ((m(C_6\text{-}C_{14})_{n\text{-alcanes}} + m(C_6\text{-}C_{14})_{iso\text{-alcanes}} +$$

$$m(C_6\text{-}C_{14})_{cyclo\text{-alcanes}} + m(C_{14+})_{n\text{-alcanes}} + m(C_{14+})_{iso\text{-alcanes}} + m(C_{14+})_{cyclo\text{-alcanes}}))_{aromatiques}$$

Le signe m indiquant la masse.
La masse des éléments des classes 3 à 10, issue de la pyrolyse des hydrocarbures de la famille résines correspond à la quantité d'hydrocarbures saturés initialement liés aux hydrocarbures de cette famille résines. Cette masse est notée, $(M)_{saturés})_{reshes}$, elle correspond à la fraction biodégradable issue des résines :

$$(M)_{saturés})_{résines} = m(C_1\text{-}C_4) + ((m(C_6\text{-}C_{14})_{n\text{-alcanes}} + m(C_6\text{-}C_{14})_{iso\text{-alcanes}} + m(C_6\text{-}C_{14})_{cyclo\text{-alcanes}}$$

$$+ m(C_{14+})_{n\text{-alcanes}} + m(C_{14+})_{iso\text{-alcanes}} + m(C_{14+})_{cyclo\text{-alcanes}}))_{résines}$$

La masse des éléments des classes 3 à 10, issue de la pyrolyse des hydrocarbures de la famille asphaltènes correspond à la quantité d'hydrocarbures saturés initialement liés aux hydrocarbures de cette famille asphaltènes. Cette masse est notée, $(M)_{saturés})_{asphaltènes}$, elle correspond à la fraction biodégradable issue des asphaltènes :

$$(M)_{saturés})_{asphaltènes} = m(C_1\text{-}C_4) + ((m(C_6\text{-}C_{14})_{n\text{-alcanes}} + m(C_6\text{-}C_{14})_{iso\text{-alcanes}} +$$

$$m(C_6\text{-}C_{14})_{cyclo\text{-alcanes}} + m(C_{14+})_{n\text{-alcanes}} + m(C_{14+})_{iso\text{-alcanes}} + m(C_{14+})_{cyclo\text{-alcanes}}))_{asphaltènes}$$

Puis les quantités d'hydrocarbures saturés sont calculées pour 100% de conversion, des $C_{14+}$ aromatiques, résines, asphaltènes ou NSOs.

## Applications

Détermination du taux de biodégradation / calcul des pertes en hydrocarbures

Le taux de biodégradation d'une huile mesure l'importance de la biodégradation, c'est-à-dire la quantité d'hydrocarbures que les bactéries ont métabolisé depuis la genèse de l'huile. Pour déterminer ce taux, il est préférable de posséder une huile non biodégradée, ou à défaut, une huile peu biodégradée. Selon, l'invention, pour déterminer le taux de biodégradation d'une huile, la méthode comporte les étapes suivantes :

1. on prélève au moins deux échantillons dans un gisement pétrolier.
2. on détermine l'échantillon le moins biodégradé.
3. on détermine la quantité d'hydrocarbures saturés liés aux familles aromatiques de plus de 14 atomes de carbone ($C_{14+}$ aromatiques), résines et asphaltènes dans les deux échantillons.
4. on en déduit le taux de biodégradation de l'échantillon le plus biodégradé

*Diagnostic de biodégradation sur les échantillons étudiés*

On dispose d'une série d'au moins 2 échantillons que l'on classe par biodégradation croissante. Pour ce faire on utilise une technique connue des spécialistes. Par exemple, on peut utiliser la méthode décrite dans le document suivant :

Peters KE, Walters CC, Moldowan JM, 2005, The Biomarker Guide, Cambridge University Press, Cambridge.

Si un des échantillons est estimé non biodégradé, alors la distribution des hydrocarbures saturés représente l'ensemble des molécules non biodégradées. Sinon, lorsque l'échantillon est déjà altéré par biodégradation, les proportions de ces différentes classes chimiques sont modifiées et ne représentent donc pas le profil des hydrocarbures saturés initiaux avant biodégradation. On peut toutefois obtenir un taux relatif de biodégradation (par rapport à l'huile la moins biodégradée).

On peut alors déterminer les quantités, c'est-à-dire les masses, de $C_6\text{-}C_{14}$ *n*-alcanes, $C_6\text{-}C_{14}$ *iso*-alcanes et $C_6\text{-}C_{14}$ *cyclo*-alcanes ainsi que les quantités de $C_{14+}$ *n*-alcanes, $C_{14+}$ *iso*-alcanes et $C_{14+}$ *cyclo*-alcanes. La quantité totale des hydrocarbures est appelée "fraction potentiellement biodégradable". Elle est notée *PBF*. Ainsi :

$$PBF = m(C_6\text{-}C_{14})_{n\text{-alcanes}} + m(C_6\text{-}C_{14})_{iso\text{-alcanes}} + m(C_6\text{-}C_{14})_{cyclo\text{-alcanes}}$$

$$+ m(C_{14+})_{n\text{-alcanes}} + m(C_{14+})_{iso\text{-alcanes}} + m(C_{14+})_{cyclo\text{-alcanes}}$$

où le signe m indique la masse.

Ensuite, on détermine les quantités d'hydrocarbures saturés liés aux familles aromatiques de plus de 14 atomes de carbone ($C_{14+}$ aromatiques), résines et asphaltènes, pour les deux échantillons. Ceci est réalisé au moyen de la méthode selon l'invention, décrite précédemment. On détermine ainsi :

- pour l'échantillon biodégradé :

  - $(M)_{saturés})_{aromatiques}$
  - $(M)_{saturés})_{résines}$
  - $(M)_{saturés})_{asphaltènes}$

- pour l'échantillon non biodégradé :

    • $((M_{100})_{saturés})_{aromatiques})$

    • $(M_{100})_{saturés})_{résines})$

    • $(M_{100})_{saturés})_{asphaltènes})$

L'indice 100 indique qu'il s'agit de la masse biodégradable de l'huile non biodégradée. Il s'agit, pour chaque masse, de la valeur maximale que peut atteindre la valeur $M$.

L'indice $x$ indique qu'il s'agit de la masse biodégradable de l'huile la moins biodégradée ($x=100$ correspond à une huile non biodégradée).

On peut alors calculer une fraction biodégradable, notée BF : $BF = M/M_{100}$. Et l'on calcule ainsi :

    • $(BF)_{saturés})_{aromatiques}$

    • $(BF)_{saturés})_{résines}$

    • $(BF)_{saturés})_{asphaltènes}$

Lorsque les huiles sont altérées par biodégradation, les valeurs de M sont égales ou inférieures aux valeurs $M_{100}$ suivant l'intensité de la biodégradation et la famille chimique considérée. Ainsi la différence entre $M_{100}$ de l'huile non biodégradée avec la valeur de M mesuré dans une huile biodégradée est égale à une partie des pertes en hydrocarbures par altération microbienne. Elle correspond en effet, à la perte des structures saturées liées aux structures aromatiques, résines et asphaltène. Ainsi :

La perte des structures saturées dans la famille $C_{14+}$ aromatiques d'une huile non biodégradée et d'une huile biodégradée est :

$$(\Delta_{saturés})_{aromatiques} = ((M_{100})_{saturés})_{aromatiques}) - ((M)_{saturés})_{aromatiques})$$

La perte des structures saturées dans la famille résine d'une huile non biodégradée et d'une huile biodégradée est :

$$(\Delta_{saturés})_{résines} = ((M_{100})_{saturés})_{résines}) - ((M)_{saturés})_{résines})$$

La perte des structures saturées dans la famille asphaltène d'une huile non biodégradée et d'une huile biodégradée est :

$$(\Delta_{saturés})_{asphaltènes} = ((M_{100})_{saturés})_{asphaltènes}) - ((M)_{saturés})_{asphaltènes})$$

Au cours de la biodégradation de la famille $C_{14+}$ aromatiques, il est possible qu'une partie des molécules aromatiques associés aux structures saturées soient perdues. Afin de quantifier ces pertes, il est possible de quantifier les proportions relatives des structures aromatiques de 1 (1 N) à plusieurs cycles (xN) dans les familles $C_{14+}$ aromatiques des huiles non biodégradées et des huiles biodégradées. Cette quantification est réalisée par des techniques connues des spécialistes, comme par exemple la spectrométrie de masse (analyse par la méthode de Fischer), la RMN 2D, la GC 2D et des fractionnements par chromatographie en phase liquide. On calcule ensuite la perte en hydrocarbures aromatiques dans la famille $C_{14+}$ aromatiques de la manière suivante :

$$(\% \ C_{14+} \ aromatiques)_{non \ bio} = ((\Sigma \ (\% \ (1N) + \% \ (2N) + \% \ (3N) + \ ....\%(6N))_{non \ bio}$$

$$(\% \; C_{14+} \; \text{aromatiques})_{bio} = ((\Sigma \; (\% \; (1N) + \% \; (2N) + \% \; (3N) + ....\%(6N))_{bio}$$

$$(\Delta_{aromatiques})_{aromatiques} = ((\Sigma \; (\% \; (1N) + \% \; (2N) + \% \; (3N) + ....\%(6N))_{non \; bio} - ((\Sigma \; (\% \; (1N) +$$

$$\% \; (2N) + \% \; (3N) + ....\%(6N))_{bio}$$

L'indice "bio" indique que l'on analyse une huile biodégradée, et l'indice "non bio" indique une huile non biodégradée. Pour les résines et les asphaltènes, il n'est pas possible d'estimer le $(\Delta_{aromatiques})_{résines}$ et le $(\Delta_{aromatiques})_{asphaltènes}$.

La perte totale en hydrocarbures saturés $(\Delta_{saturés})_{total}$ pour les $C_{14+}$ aromatiques, résines et asphaltènes dans une huile biodégradée, est donnée par la somme :

$$(\Delta_{saturés})_{total} = (\Delta_{saturés})_{aromatiques} + (\Delta_{saturés})_{résines} + (\Delta_{saturés})_{asphaltènes}$$

Pour les pertes en hydrocarbures aromatiques, elle ne peut être réalisée que sur famille $C_{14+}$ aromatiques des huiles.

$$(\Delta_{aromatiques})_{total} = (\Delta_{aromatiques})_{aromatiques}$$

Et donc la perte totale en hydrocarbures $(\Delta_{hc})_{total}$ due à la biodégradation est :

$$(\Delta_{hc})_{total} = (\Delta_{saturés})_{total} + (\Delta_{aromatiques})_{aromatiques}$$

Cette perte totale correspond au taux de biodégradation de l'huile. Elle peut ensuite être directement corrélée au degré API des huiles étudiées.

Détermination du degré API ou la fraction biodégradable d'une huile

Il est ensuite possible de déterminer dans quel intervalle de PBF, les valeurs respectives des BF des trois familles restent maximales. Ainsi, dans le cas d'huiles biodégradées, il est possible de retrouver les valeurs de PBF en utilisant les valeurs $BF_{100}$ d'une ou des trois familles $C_{14+}$ aromatiques, résines et asphaltènes.

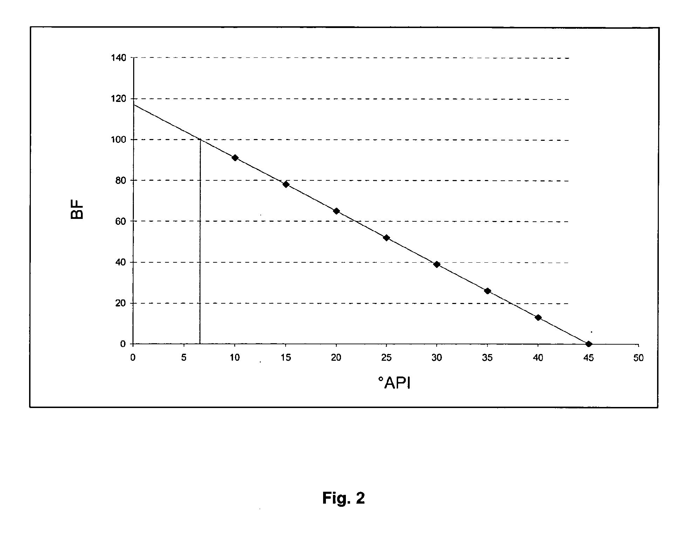

La méthodologie permet donc de déterminer des PBF (de l'huile non biodégradée si celle-ci n'est pas disponible) et le degré API de l'huile la plus biodégradée qui serait potentiellement trouvée dans ce bassin. Il suffit pour cela de placer les BF des échantillons analysés en fonction de leur degré API sur une figure et de prolonger d'une part la courbe qui pourrait être par exemple une droite, jusqu'à ce qu'elle coupe l'axe des abscisses (c'est le PBI) et d'autre part de prolonger cette même courbe ou par exemple une droite jusqu'à la valeur de BF égale à 100% et dont la valeur de l'abscisse correspond au °API le plus bas que peut atteindre cette huile (cf. figure 2).

Sur la figure 2, l'axe des abscisses représente le degré API et l'axe des ordonnées représente une fraction biodégradable BF d'une des familles $C_{14+}$ aromatiques, résines et asphaltènes. On peut également utiliser la somme de ces trois fractions biodégradables BF.

En appliquant la méthode selon l'invention on peut déterminer les fractions biodégradables BF pour les $C_{14+}$ aromatiques, résines et asphaltènes dans une huile biodégradée. On connaît par ailleurs des méthodes pour déterminer le degré API d'une huile. On peut alors positionner les échantillons d'huile sur le graphique de la figure 2 (les losanges sur la figure 2).

Puis, pour chaque nouvel échantillon extrait du gisement pétrolier, on mesure son degré API, et au moyen d'une interpolation linéaire (la droite passant par les losanges sur la figure 2), ou de toute autre relation, on détermine directement les fractions biodégradables BF pour les $C_{14.}$ aromatiques, résines et asphaltènes (en utilisant un graphique et une interpolation pour chacune de ces fractions). Ainsi, il est possible d'assigner à une huile d'un champ donné, une valeur de fraction biodégradable BF, et d'en déduire sa qualité économique. A l'échelle d'un bassin pour lequel un grand nombre de données de degré API est disponible, il est alors possible d'établir des cartes de fraction biodégradable BF, et de déterminer des tendances régionales de biodégradation, et d'ainsi, prédire la localisation de puits de forage les plus rentables économiquement.

Ainsi, on peut déterminer le degré API ou la fraction biodégradable d'une huile biodégradée extraite d'un gisement pétrolier, en réalisant une phase de calibration, au cours de laquelle :

- on prélève au moins deux échantillons d'huile dans le gisement pétrolier ;

- on détermine pour chacun de ces échantillons d'huile la quantité d'hydrocarbures saturés liés à chacune des familles (hydrocarbures aromatiques de plus de quatorze atomes de carbone, résines et asphaltènes) ;

- on détermine pour chacun de ces échantillons d'huile un degré API ;

- on détermine une relation entre les quantité d'hydrocarbures saturés liés et les degrés API ;

Puis, une fois la relation établie, on peut -déterminer le degré API de tout échantillon d'huile extrait du gisement pétrolier, en déterminant les quantités d'hydrocarbures saturés liés à chacune des familles (hydrocarbures aromatiques de plus de quatorze atomes de carbone, résines et asphaltènes), et en appliquant ladite relation.

Inversement, on peut déterminer la fraction biodégradable de tout échantillon d'huile extrait du gisement pétrolier, en déterminant le degré API, et en appliquant la relation.

_Estimation de la réactivité des trois familles $C_{14+}$ aromatiques, résines et asphaltènes_

_vis-à-vis de la biodégradation._

Les variations relatives des BF pour chacune des trois familles $C_{14+}$ aromatiques, résines et asphaltènes en fonction de la biodégradation déterminent la bio réactivité relative de ces trois familles.

La méthode selon l'invention, pour déterminer la quantité d'hydrocarbures saturés liés aux familles aromatiques de plus de 14 atomes de carbone ($C_{14+}$ aromatiques), résines et asphaltènes, permet par ces applications, de déterminer les conditions d'exploitation d'un réservoir pétrolier. En effet, le taux de biodégradation (la fraction biodégradable) des huiles extraites du gisement, permet de dresser des cartes de zones de qualité d'huile. Les forages seront ainsi plus précisément déterminés, et les conditions de forage pour la production ou l'injection pourront être ainsi définis.

La méthode peut également s'appliquer à l'évaluation de la biodégradation des hydrocarbures dans des sites pollués ou dans des expériences de simulation de la biodégradation au laboratoire.

**Revendications**

1.   Méthode d'analyse d'un échantillon pétrolier contenant au moins une famille d'hydrocarbures aromatiques de plus de quatorze atomes de carbone, une famille de résines, et une famille d'asphaltènes, dans laquelle on détermine une quantité d'hydrocarbures saturés liés à chacune desdites familles, en réalisant les étapes suivantes :

      - on réalise une séparation desdites familles au sein dudit échantillon pétrolier ;
      - on génère les hydrocarbures saturés liés à chacune desdites familles au moyen de pyrolyses en milieu fermé, chacune des trois familles étant pyrolysées de façon indépendante, avec des gammes de températures de pyrolyse et des gammes de temps de pyrolyse déterminées pour effectuer un craquage desdites familles générant les hydrocarbures saturés initialement liés aux hydrocarbures de chacune des trois familles ;
      - on réalise une séparation des hydrocarbures saturés à la fin de chaque pyrolyse ;
      - on détermine la quantité d'hydrocarbures saturés liés à chacune desdites familles en dosant les hydrocarbures saturés ainsi séparés.

2.   Méthode selon la revendication 1, dans laquelle lesdites gammes de températures de pyrolyse sont comprises entre 300 et 350°C, et lesdites gammes de temps de pyrolyse sont supérieures à une heure.

3.   Méthode selon l'une des revendications précédentes, dans laquelle lesdites gammes de températures de pyrolyse et de temps de pyrolyse sont déterminées à partir de paramètres cinétiques (E, A) de réactions chimiques affectant lesdites familles au cours d'un craquage d'une huile, au moyen des étapes suivantes pour chaque famille :

- on détermine une vitesse de dégradation de ladite famille pour une température donnée en utilisant la loi d'Arrhenius, et on en déduit un taux de conversion pour différents temps de pyrolyse ;
- on sélectionne les gammes de températures de pyrolyse et de temps de pyrolyse qui conduisent à un taux de conversion de 100%.

4. Méthode selon l'une des revendications 1 et 2, dans laquelle on ne génère qu'une partie des hydrocarbures saturés initialement liés aux hydrocarbures de chacune des trois familles, lesdites gammes de températures de pyrolyse et de temps de pyrolyse sont déterminées à partir de paramètres cinétiques (E, A) de réactions chimiques affectant lesdites familles au cours d'un craquage d'une huile, au moyen des étapes suivantes pour chaque famille :

- on détermine une vitesse de dégradation de ladite famille pour une température donnée en utilisant la loi d'Arrhenius, et on en déduit un taux de conversion pour différents temps de pyrolyse ;
- on sélectionne les gammes de températures de pyrolyse et de temps de pyrolyse qui conduisent à un taux de conversion suffisant pour récupérer des quantités d'hydrocarbures saturés mesurables,
- on calcul, grâce au taux de conversion connus pour les différentes conditions de pyrolyse, la quantité maximale d'hydrocarbures saturés pour 100% de conversion.

5. Méthode selon l'une des revendications précédentes, dans laquelle le dosage est réalisé en utilisant les classes chimiques suivantes :

| Classe 1 | $CO_2$ |
|---|---|
| Classe 2 | $H_2S$ |
| Classe 3 | $C_1$ |
| Classe 4 | $C_2$-$C_4$ |
| Classe 5 | $C_6$-$C_{14}$ *n*-alcanes |
| Classe 6 | $C_6$-$C_{14}$ *iso*-alcanes |
| Classe 6 | $C_6$-$C_{14}$ *cyclo*-alcanes |
| Classe 7 | $C_6$-$C_{14}$ aromatiques |
| Classe 8 | $C_{14+}$ *n*-alcanes |
| Classe 9 | $C_{14+}$ *iso*-alcanes |
| Classe 10 | $C_{14+}$ *cyclo*-alcanes |
| Classe 11 | $C_{14+}$ aromatiques |
| Classe 12 | Résines |
| Classe 13 | Asphaltènes |
| Classe 14 | Résidu insoluble |

6. Méthode selon l'une des revendications précédentes, dans laquelle on détermine une fraction biodégradable d'une huile biodégradée extraite d'un gisement pétrolier, en réalisant les étapes suivantes :

- on prélève un échantillon d'huile non biodégradée dans ledit gisement pétrolier ;
- on détermine, pour lesdits échantillon d'huile non biodégradée et biodégradée, la quantité d'hydrocarbures saturés liés à chacune desdites familles, hydrocarbures aromatiques de plus de quatorze atomes de carbone, résines et asphaltènes ;
- on détermine la fraction biodégradable de l'huile biodégradée en calculant le rapport entre la quantité d'hydrocarbures saturés de l'échantillon biodégradé et la quantité d'hydrocarbures saturés de l'échantillon non biodégradé.

7. Méthode selon la revendication 6, dans laquelle on détermine un degré API d'une huile biodégradée extraite d'un gisement pétrolier, en réalisant les étapes suivantes :

- on réalise une phase de calibration, au cours de laquelle :
- on prélève au moins deux échantillons d'huile dans ledit gisement pétrolier ;
- on détermine pour chacun desdits échantillons d'huile une quantité d'hydrocarbures saturés liés à chacune desdites familles, hydrocarbures aromatiques de plus de quatorze atomes de carbone, résines et asphaltènes ;
- on détermine pour chacun desdits échantillons d'huile un degré API ;
- on détermine une relation entre lesdites quantité d'hydrocarbures saturés liés et les degrés API ;
- on détermine le degré API de tout échantillon d'huile extrait du gisement pétrolier, en déterminant des quantités d'hydrocarbures saturés liés à chacune desdites familles, hydrocarbures aromatiques de plus de quatorze atomes de carbone, résines et asphaltènes, et en appliquant ladite relation.

8. Méthode selon la revendication 6, dans laquelle on détermine une fraction biodégradable d'une huile biodégradée extraite d'un gisement pétrolier, en réalisant les étapes suivantes :

- on réalise une phase de calibration, au cours de laquelle :
- on prélève au moins deux échantillons d'huile dans ledit gisement pétrolier ;
- on détermine pour chacun desdits échantillons d'huile une quantité d'hydrocarbures saturés liés à chacune desdites familles, hydrocarbures aromatiques de plus de quatorze atomes de carbone, résines et asphaltènes ;
- on détermine pour chacun desdits échantillons d'huile un degré API ;
- on détermine une relation entre lesdites quantité d'hydrocarbures saturés liés et les degrés API ;
- on détermine la fraction biodégradable de tout échantillon d'huile extrait du gisement pétrolier, en déterminant le degré API, et en appliquant ladite relation.

ECH

$C_5$-$C_{14}$ SAT
$C_6$-$C_{14}$ AR

$C_{14+}$SAT

$C_{14+}$AR

RES

ASP

PYR
$T_a$, $t_a$

PYR
$T_r$, $t_r$

PYR
$T_{as}$, $t_{as}$

$(M)_{saturés})_{aromatiques}$
$(M)_{aromatiques})_{aromatiques}$

$(M)_{saturés})_{asphalténes}$

$(M)_{saturés})_{résines}$

**Fig. 1**

**Fig. 2**

Europäisches
Patentamt

European
Patent Office

Office européen
des brevets

**RAPPORT DE RECHERCHE EUROPEENNE**

Numéro de la demande

EP 10 29 0439

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | BOWDEN S A ET AL: "Compositional differences in biomarker constituents of the hydrocarbon, resin, asphaltene and kerogen fractions: An example from the Jet Rock (Yorkshire, UK)", ORGANIC GEOCHEMISTRY, vol. 37, no. 3, 1 mars 2006 (2006-03-01), pages 369-383, XP025088459, ISSN: 0146-6380, DOI: 10.1016/J.ORGGEOCHEM.2005.08.024 [extrait le 2006-03-01] * le document en entier * ----- | 1-6 | INV. G01N33/28 G01N31/12 |
| Y,D | BEHAR F ET AL: "Elaboration of a new compositional kinetic schema for oil cracking", ORGANIC GEOCHEMISTRY, vol. 39, no. 6, 1 juin 2008 (2008-06-01), pages 764-782, XP022679438, ISSN: 0146-6380, DOI: 10.1016/J.ORGGEOCHEM.2008.03.007 [extrait le 2008-03-25] * page 767, colonne de gauche, alinéa 3 - page 770, colonne de droite, alinéa 1 * * page 774, colonne de droite, alinéa 3 - page 780, colonne de droite, alinéa 2 * ----- | 1-6 | |
| A | FR 2 888 251 A1 (INST FRANCAIS DU PETROLE [FR]) 12 janvier 2007 (2007-01-12) * page 8 - page 15; figures * ----- | 1,6 | DOMAINES TECHNIQUES RECHERCHES (IPC) G01N C12M C12Q |

Le présent rapport a été établi pour toutes les revendications

| Lieu de la recherche | Date d'achèvement de la recherche | Examinateur |
|---|---|---|
| Munich | 30 novembre 2010 | Johnson, Keith |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 10 29 0439

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

30-11-2010

| Document brevet cité au rapport de recherche | Date de publication | Membre(s) de la famille de brevet(s) | Date de publication |
|---|---|---|---|
| FR 2888251 A1 | 12-01-2007 | DE 102006031200 A1<br>US 2007007203 A1 | 18-01-2007<br>11-01-2007 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **Connan et al.** Advances in Organic Geochemistry. Pegamon Press, 1979, 1-17
- **Peters ; Moldowan.** The biomarker guide. Prentice Hall, 1993
- **Peters et al.** the biomarker guide : biomarkers and isotopes in petroleum exploration and earth history. Cambridge University Press, 2005
- **Behar F. et al.** Elaboration of a new compositional kinetic schema for oil cracking. *Organic Geochemistry,* 2008, vol. 39, 764-782
- **Peters KE ; Walters CC ; Moldowan JM.** The Biomarker Guide. Cambridge University Press, 2005